# EUROPÄISCHE PATENTSCHRIFT

(11) **EP 0 872 480 B1**
(45) Veröffentlichungstag und Bekanntmachung des Hinweises auf die Patenterteilung: **19.09.2001**
(21) Anmeldenummer: 98106760.6
(22) Anmeldetag: 14.04.1998
(51) Int. Cl.: C07D 271/113

(54) **Verfahren zur Herstellung von substituierten Oxadiazolonen**
Process for the preparation of substituted oxadiazolones
Procédé pour la préparation des oxadiazolones substituées

(30) Priorität: 15.04.1997 US 842585
(43) Veröffentlichungstag der Anmeldung: 21.10.1998
(73) Patentinhaber: Bayer Corporation, Pittsburgh, PA 15205-9741 (US); BAYER AG, 51368 Leverkusen (DE)
(72) Erfinder: Diehr, Hans-Joachim Dr., 42329 Wuppertal (DE); Lantzsch, Reinhard Dr., 42115 Wuppertal (DE); Applegate, Jacqueline M. Dr., 51377 Leverkusen (DE); Jelich, Klaus Dr., Overland Park KS 66223 (US)
(74) Vertreter: Linkenheil, Dieter

(56) Entgegenhaltungen:
- EP-A- 0 130 447
- EP-A- 0 270 061
- EP-A- 0 301 946
- J GANTE: "Über neuartige Azapeptide" CHEMISCHE BERICHTE, Bd. 98, 1965, Seiten 540-544, XP002070635
- A.KURTZ ET AL: "Synthesis and properties of ethyl 1-Acetyl-2-benzyl carbazate" JOURNAL OF ORGANIC CHEMISTRY, Bd. 26, 1961, Seiten 1843-1846, XP002070636
- T RABINI ET AL: "Synthesis of Monoacyl Hydrazides" JOURNAL OF ORGANIC CHEMISTRY, Bd. 30, 1965, Seiten 2486-2488, XP002070637

## Beschreibung

Die Erfindung betrifft ein neues Verfahren zur Herstellung von substituierten Oxadiazolonen, welche als Zwischenprodukte für die Herstellung von herbizid wirksamen Verbindungen bekannt sind.

Es ist bekannt, daß man bestimmte substituierte Oxadiazolone, wie z.B. 5-Methyl-1,3,4-oxadiazol-2(3H)-on, erhält, wenn man acylierte Hydrazine, wie z.B. Acethydrazid, mit Phosgen umsetzt (vgl. Bull. Chem. Soc. Japan 44 (1971), 870; Chem. Ber. 98 (1965), 540-545; Helv. Chim. Acta 55 (1972), 1174-1178; J. Org. Chem. 26 (1961), 1843-1846; EP 301946; EP 321833).

Die hierfür als Ausgangsstoffe benötigten acylierten Hydrazine werden im allgemeinen in einer vorgeschalteten Umsetzungsstufe aus entsprechenden Carbonsäuren und Hydrazin(-hydrat) in Gegenwart von Katalysatoren hergestellt (vgl. J. Org. Chem. 30 (1965), 2486-2488; GB 1396615; EP 653419).

Die Umsetzungen von Carbonsäuren mit Hydrazin(-hydrat) werden jedoch in Gemischen aus verschiedenen Lösungsmitteln durchgeführt, was für technische Belange ungünstig ist, da diese Lösungsmittel nicht ohne weiteres im Kreis geführt oder in reiner Form zurückgewonnen werden können. Vor der anschließenden Phosgenierung müssen die acylierten Hydrazine nach dem Stand der Technik in einer aufwendigen Prozedur isoliert und getrocknet werden. Es bestand daher Bedarf an einem für technische Belange besser geeigneten Herstellungsverfahren für substituierte Oxadiazolone.

Es wurde nun gefunden, daß man substituierte Oxadiazolone der allgemeinen Formel (I) 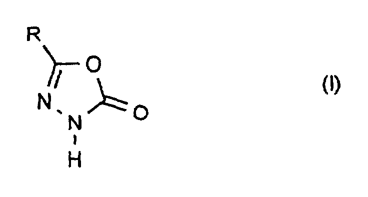 in welcher
- R: für gegebenenfalls durch Halogen oder C₁-C₄-Alkoxy substituiertes geradkettiges oder verzweigtes Alkyl mit 1 bis 6 Kohlenstoffatomen steht,

in guten Ausbeuten und in hoher Reinheit erhält, wenn man
in einer ersten Stufe Carbonsäuren der allgemeinen Formel (II)

   R-COOH (II)
in welcher
   - R: die oben angegebene Bedeutung hat,
mit Hydrazinhydrat in Gegenwart eines Katalysators und gegebenenfalls in Gegenwart eines inerten Verdünnungsmittels in Abwesenheit eines Alkohols bei Temperaturen zwischen 0°C und 150°C unter Abscheiden von Wasser umsetzt und die hierbei gebildeten Carbonsäurehydrazide der allgemeinen Formel (III)

   R-CO-NH-NH₂ (III)
in welcher
   - R: die oben angegebene Bedeutung hat,
in einer zweiten Stufe im gleichen Reaktionsmedium - d.h. ohne Zwischenisolierung - mit Phosgen (COCl₂) bei Temperaturen zwischen 20°C und 120°C umsetzt.

Es ist als ausgesprochen überraschend anzusehen, daß die Umsetzung von Carbonsäuren mit Hydrazinhydrat an Stelle der üblichen Alkohol-Benzol- oder Alkohol-Toluol-Gemische auch in inerten Lösungsmitteln ohne Verwendung von Alkoholen-oder ganz ohne gesonderte Lösungsmittel - problemlos durchgeführt werden kann und die nachfolgende Umsetzung mit Phosgen auch ohne Zwischenisolierung der acylierten Hydrazine und ohne Lösungsmittelwechsel mit sehr gutem Erfolg verläuft. Das erfindungsgemäße Verfahren stellt somit eine wertvolle Bereicherung des Standes der Technik dar.

Das erfindungsgemäße Verfahren betrifft insbesondere die Herstellung von Verbindungen der Formel (I), in welcher
- R: für jeweils gegebenenfalls durch Fluor, Chlor, Methoxy oder Ethoxy substituiertes Methyl, Ethyl, n- oder i-Propyl, n-, i-, s- oder t-Butyl steht.

Verwendet man beispielsweise Essigsäure und Hydrazinhydrat als Ausgangsstoffe, so kann das erfindungsgemäße Verfahren durch das folgende Formelschema skizziert werden:

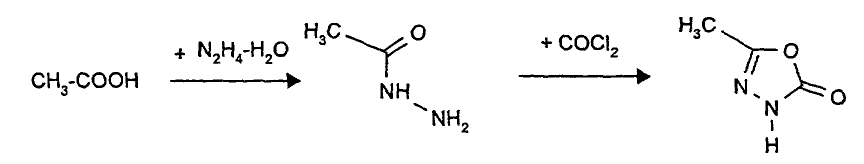

Die beim erfindungsgemäßen Verfahren als Ausgangsstoffe zu verwendenden Carbonsäuren sind durch die Formel (II) allgemein definiert. In der Formel (II) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde.

Die Ausgangsstoffe der Formel (II) sind bekannte organische Chemikalien.

Das erfindungsgemäße Verfahren wird in Gegenwart eines Katalysators durchgeführt. Als Katalysatoren sind hierbei besonders Stoffe geeignet, welche Kondensationsreaktionen - d.h. unter Wasserabspaltung verlaufende Umsetzungen - von Carbonsäuren mit Alkoholen, Aminen, Hydrazinen etc. beschleunigen. Hierzu gehören beispielsweise (ggf. aktiviertes) Aluminiumoxid, Titandioxid, Zirconiumoxid, Aluminium-, Titan- oder Zirconium-alkoholate oder -halogenid-alkoholate, wie z.B. Aluminium-tri-i-propylat, Titan-tetramethylat, Titan-tetraethylat, Titan-tetra-n-propylat, Titan-tetra-i-propylat, Titan-tetra-n-butylat, Titan-tetra-i-butylat oder Titan-tetra-s-butylat.

Als besonders bevorzugte Katalysatoren seien Aluminiumoxid, Titandioxid, Titan-tetra-n-propylat und Titan-tetra-i-propylat genannt. Die genannten Metalloxide sind überraschenderweise auch dann aktiv, wenn sie als Hydrate vorliegen, d.h. Wasser enthalten. Der Katalysator kann also gegebenenfalls auch zurückgewonnen und "im Kreis geführt" werden.

Das erfindungsgemäße Verfahren wird gegebenenfalls unter Verwendung eines inerten Verdünnungsmittels durchgeführt. Als inerte Verdünnungsmittel sind in diesem Zusammenhang vor allem organische Lösungsmittel zu verstehen, welche keine leicht abspaltbaren Wasserstoffatome enthalten. Hierzu gehören vorzugsweise aliphatische, alicyclische oder aromatische, gegebenenfalls halogenierte Kohlenwasserstoffe, wie beispielsweise Benzin, Ligroin, Benzol, Toluol, Xylol, Chlorbenzol, Dichlorbenzol, Hexan, Heptan, Octan, Cyclohexan, Methylcyclohexan; Ether, wie beispielsweise Methyl-t-butyl-ether, Diisopropylether, Diisobutylether, Dioxan, Tetrahydrofuran oder Ethylenglykoldimethyl- oder -diethylether; Nitrile, wie Acetonitril, Propionitril oder Butyronitril.

Aromatische Kohlenwasserstoffe, wie z.B. Toluol, Xylol oder Chlorbenzol, werden als Verdünnungsmittel beim erfindungsgemäßen Verfahren besonders bevorzugt eingesetzt.

Die Reaktionstemperaturen können bei der Durchführung des erfindungsgemäßen Verfahrens in einem größeren Bereich variiert werden. Im allgemeinen arbeitet man - wie oben angegeben - in der ersten Stufe bei Temperaturen zwischen 0°C und 150°C, vorzugsweise zwischen 80°C und 130°C, in der zweiten Stufe zwischen 20°C und 120°C, vorzugsweise zwischen 40°C und 90°C.

Das erfindungsgemäße Verfahren wird im allgemeinen unter Normaldruck durchgefiihrt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens setzt man in der ersten Stufe pro Mol Carbonsäure der Formel (II) im allgemeinen 0,9 bis 2,0 Mol, vorzugsweise 1,0 bis 1,2 Mol, Hydrazinhydrat und 1 bis 50 mMol, vorzugsweise 2 bis 30 mMol, Katalysator, und in der zweiten Stufe 1 bis 5 Mol, vorzugsweise 1,1 bis 2,5 Mol, Phosgen ein.

In einer bevorzugten Ausführungsform des erfindungsgemäßen Verfahrens wird die Carbonsäure der Formel (II) - gegebenenfalls zusammen mit einem Verdünnungsmittel - vorgelegt und das Hydrazinhydrat und der Katalysator werden in beliebiger Reihenfolge eindosiert. Die Reaktionsmischung wird dann bis zum Ende der Umsetzung (erste Stufe) erhitzt, wobei das freigesetzte Wasser am Wasserabscheider entfernt wird. Zur Durchführung der zweiten Stufe wird dann gegebenenfalls mit einem geeigneten organischen Lösungsmittel verdünnt und Phosgen bis zum Ende der Umsetzung eingeleitet. Anschließend wird das Lösungsmittel unter vermindertem Druck sorgfältig abdestilliert und das als Rückstand erhaltene Rohprodukt gegebenenfalls durch Vakuumdestillation gereinigt (vgl. die Herstellungsbeispiele).

Die nach dem erfindungsgemäßen Verfahren herzustellenden substituierten Oxadiazolone der Formel (I) sind bereits als Ausgangsstoffe für herbizid wirksame Verbindungen bekannt (vgl. EP 321833).

### Herstellungsbeispiele:

### Beispiel 1

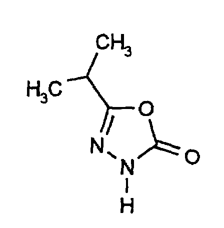

30 g (0,6 Mol) Hydrazinhydrat werden bei Raumtemperatur (ca. 20°C) unter Rühren zu einer Lösung von 44,1 g (0,5 Mol) Isobuttersäure in 90 ml Toluol tropfenweise gegeben. Dann werden bei ca. 45°C 1,42 g (5 mMol) Titan-tetra-i-propylat dazu gegeben und die Reaktionsmischung wird anschließend ca. zwei Stunden am Wasserabscheider zum Sieden erhitzt. Dabei werden ca. 23 ml Wasser abgeschieden. Das entstandene Titandioxid wird in der Siedehitze durch Filtration abgetrennt (und kann erneut als Katalysator eingesetzt werden). Das noch heiße Filtrat wird mit 400 ml Toluol verdünnt. Dann werden bei ca. 70°C bis 80°C 55,5 g Phosgen innerhalb von ca. zwei Stunden eingeleitet. Man läßt dann etwas abkühlen und destilliert unter vermindertem Druck das Lösungsmittel sorgfältig ab.

Man erhält 62,4 g (97,5% der Theorie) 5-i-Propyl-1,3,4-oxadiazol-2(3H)-on als rosastichige Flüssigkeit.

### Beispiel 2

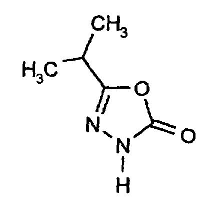

25 g (0,5 Mol) Hydrazinhydrat werden bei Raumtemperatur (ca. 20°C) unter Rühren zu einer Mischung aus 44 g (0,5 Mol) Isobuttersäure und 1,4 g (5 mMol) Titan-tetra-i-propylat tropfenweise gegeben. Die Mischung wird drei Stunden auf 100°C erhitzt, der Katalysator (wieder einsetzbar) in der Hitze durch Filtration abgetrennt und das Filtrat anschließend mit 500 ml Toluol verdünnt. Dann werden bei ca. 70°C bis 80°C 55,5 g Phosgen innerhalb von ca. zwei Stunden eingeleitet. Man läßt dann etwas abkühlen, destilliert unter vermindertem Druck das Lösungsmittel sorgfältig ab und reinigt das Produkt durch Vakuumdestillation.

Man erhält 56,3 g (88% der Theorie) 5-i-Propyl-1,3,4-oxadiazol-2(3H)-on vom Siedepunkt 108°C bis 110°C bei 3-4 mbar.

### Beispiel 3

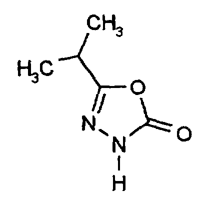

30 g (0,6 Mol) Hydrazinhydrat werden bei Raumtemperatur (ca. 20°C) unter Rühren zu einer Lösung von 44,1 g (0,5 Mol) Isobuttersäure in 90 ml Toluol tropfenweise gegeben. Dann werden bei ca. 45°C 10 g Aluminiumoxid (sauer) dazu gegeben und die Reaktionsmischung wird anschließend ca. zwei Stunden am Wasserabscheider zum Sieden erhitzt. Dabei werden ca. 23 ml Wasser abgeschieden. Die noch heiße Mischung wird zur Entfernung des Katalysators (wieder einsetzbar) filtriert und das Filtrat wird mit 400 ml Toluol verdünnt. Dann werden bei ca. 70°C bis 80°C 55,5 g Phosgen innerhalb von ca. zwei Stunden eingeleitet. Man läßt dann etwas abkühlen, filtriert dann und destilliert vom Filtrat unter vermindertem Druck das Lösungsmittel sorgfältig ab.

Man erhält 61,2 g (95,6% der Theorie) 5-i-Propyl-1,3,4-oxadiazol-2(3H)-on als rosastichige Flüssigkeit.

### Herstellung und Isolierung der Carbonsäurehydrazide der Formel (III):

### Beispiel (III-1)

(CH₃)₂CH-CO-NH-NH₂

225 g (4,4 Mol) Hydrazinhydrat werden zu einer Mischung aus 356 g (4,0 Mol) Isobuttersäure und 1 Liter Toluol gegeben. Nach Zugabe von 35,5 g (0,12 Mol) Titan-tetra-i-propylat wird das Gemisch über eine Destillationskolonne am Wasserabscheider zum Sieden erhitzt. 164 g einer "wässrigen Phase" (bestehend aus Wasser, Isopropanol und Hydrazin) werden dabei innnerhalb von ca. 5 Stunden abgeschieden ("ausgekreist"). Dann wird der Katalysator (wieder verwendbar) von der heißen Mischung durch Filtration abgetrennt. Das Filtrat kann analog zu den Beispielen 1 bis 3 in einer zweiten Stufe weiter umgesetzt werden. Zur Bestimmung der Ausbeute wird das Lösungsmittel unter vermindertem Druck sorgfältig abdestilliert.

Man erhält 397 g Isobuttersäurehydrazid (Gehalt: 98,8%, Ausbeute: 96% der Theorie) als festen, farblosen Rückstand.

### Beispiel (III-2)

(CH₃)₂CH-CO-NH-NH₂

225 g (4,4 Mol) Hydrazinhydrat werden zu einer Mischung aus 356 g (4,0 Mol) Isobuttersäure und 1 Liter Toluol gegeben. Nach Zugabe von 21,1 g (0,12 Mol) Titan-Katalysator (vorher separat durch Umsetzung von Titan-tetra-n-propylat mit Wasser hergestellt) wird das Gemisch über eine Destillationskolonne am Wasserabscheider zum Sieden erhitzt. Nach etwa einer Stunde wird der Katalysator (wieder verwendbar) in der Siedehitze durch Filtration abgetrennt (20,4 g, 97% Rückgewinnung) und mit heißem Toluol nachgewaschen. Das Filtrat kann analog zu den Beispielen 1 bis 3 in einer zweiten Stufe weiter umgesetzt werden. Zur Bestimmung der Ausbeute wird das Filtrat auf ca. 10°C abgekühlt und das kristallin angefallene Produkt durch Absaugen isoliert.

Man erhält 408 g Isobuttersäurehydrazid (Gehalt: 98,4%, Ausbeute: 98% der Theorie).

### Beispiel (III-3)

(CH₃)₂CH-CO-NH-NH₂

214,5 g (4,2 Mol) Hydrazinhydrat werden zu einer Mischung aus 356 g (4,0 Mol) Isobuttersäure und 1 Liter Toluol gegeben. Nach Zugabe von 12,8 g (0,16 Mol) Titandioxid wird das Gemisch über eine Destillationskolonne am Wasserabscheider zum Sieden erhitzt. Nach ca. 90 Minuten (Abscheidung von 116 g "Wasserphase") wird der Katalysator (wieder verwendbar) von der heißen Lösung durch Filtration abgetrennt (12,4 g, 97% Rückgewinnung) und mit heißem Toluol nachgewaschen. Das Filtrat kann analog zu den Beispielen 1 bis 3 in einer zweiten Stufe weiter umgesetzt werden. Zur Bestimmung der Ausbeute wird das Lösungsmittel unter vermindertem Druck sorgfältig abdestilliert.

Man erhält 410 g Isobuttersäurehydrazid (Gehalt: 97,9%, Ausbeute: 98% der Theorie).

## Patentansprüche

1. Verfahren zur Herstellung von substituierten Oxadiazolen der allgemeinen Formel (I), 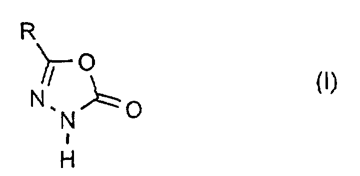 in welcher
R für gegebenenfalls durch Halogen oder C₁-C₄-Alkoxy substituiertes geradkettiges oder verzweigtes Alkyl mit 1 bis 6 Kohlenstoffatomen steht,
**dadurch gekennzeichnet, daß** man
in einer ersten Stufe Carbonsäuren der allgemeinen Formel (II)
R-COOH (II)
in welcher
R die oben angegebene Bedeutung hat,
mit Hydrazinhydrat in Gegenwart eines Katalysators und gegebenenfalls in Gegenwart eines inerten Verdünnungsmittels in Abwesenheit eines Alkohols bei Temperaturen zwischen 0°C und 150°C unter Abscheiden von Wasser umsetzt und die hierbei gebildeten Carbonsäurehydrazide der allgemeinen Formel (III)
R-CO-NH-NH₂ (III)
in welcher
R die oben angegebene Bedeutung hat,
in einer zweiten Stufe im gleichen Reaktionsmedium - d.h. ohne Zwischenisolierung - mit Phosgen (COCl₂) bei Temperaturen zwischen 20°C und 120°C umsetzt.

2. Verfahren nach Anspruch 1, **dadurch gekennzeichnet, daß** in den Formeln (I), (II) und (III) der Rest
R für jeweils gegebenenfalls durch Fluor, Chlor, Methoxy oder Ethoxy substituiertes Methyl, Ethyl, n- oder i-Propyl, n-, i-, s- oder t-Butyl steht.

3. Verfahren nach Anspruch 1, **dadurch gekennzeichnet, daß** man in der ersten Verfahrensstufe bei Temperaturen zwischen 80°C und 130°C arbeitet.

4. Verfahren nach Anspruch 1, **dadurch gekennzeichnet, daß** man in der zweiten Verfahrensstufe bei Temperaturen zwischen 40°C und 90°C arbeitet.

5. Verfahren nach Anspruch 1, **dadurch gekennzeichnet, daß** man in der ersten Verfahrensstufe als Katalysator Aluminiumoxid, Titandioxid, Zirconiumoxid, ein Aluminium-, Titan- oder Zirconium-alkoholat oder -halogenid-alkoholat einsetzt.

6. Verfahren nach Anspruch 5, **dadurch gekennzeichnet, daß** man als Katalysator Aluminium-tri-i-propylat, Titan-tetramethylat, Titan-tetraethylat, Titan-tetra-n-propylat, Titan-tetra-i-propylat, Titan-tetra-n-butylat, Titan-tetra-i-butylat oder Titan-tetra-s-butylat einsetzt.

7. Verfahren nach Anspruch 5, **dadurch gekennzeichnet, daß** man als Katalysator Aluminiumoxid, Titandioxid, Titan-tetra-n-propylat oder Titan-tetra-i-propylat einsetzt.

## Claims

1. Process for preparing substituted oxadiazolones of the general formula (I) 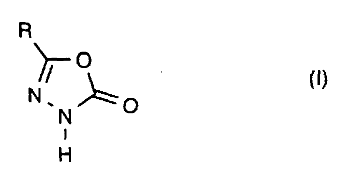 in which
R represents optionally halogen- or C₁-C₄-alkoxy-substituted straight-chain or branched alkyl having 1 to 6 carbon atoms.
**characterized in that**
in a first step carboxylic acids of the general formula (II)
R-COOH (II)
in which
R is as defined above
are reacted with hydrazine hydrate in the presence of a catalyst and, if appropriate, in the presence of an inert diluent in the absence of an alcohol at temperatures between 0°C and 150°C with elimination of water, and the resulting carboxylic hydrazides of the general formula (III)
R-CO-NH-NH₂ (III)
in which
R is as defined above are reacted in a second step in the same reaction medium - i.e. without isolation of the intermediate - with phosgene (COCl₂) at temperatures between 20°C and 120°C.

2. Process according to Claim 1, **characterized in that** in the formulae (I), (II) and (III) the radical
R represents in each case optionally fluorine-, chlorine-, methoxy- or ethoxy-substituted methyl, ethyl, n- or i-propyl, n-, i-, s- or t-butyl.

3. Process according to Claim 1, **characterized in that** the first process step is carried out at temperatures between 80°C and 130°C.

4. Process according to Claim 1, **characterized in that** the second process step is carried out at temperatures between 40°C and 90°C.

5. Process according to Claim 1, **characterized in that** the catalyst used in the first process step is aluminium oxide, titanium dioxide, zirconium oxide, an aluminium, titanium or zirconium alkoxide or an aluminium, titanium or zirconium halide alkoxide.

6. Process according to Claim 5, **characterized in that** the catalyst used is aluminium tri-i-propoxide, titanium tetramethoxide, titanium tetraethoxide, titanium tetra-n-propoxide, titanium tetra-i-propoxide, titanium tetra-n-butoxide, titanium tetra-i-butoxide or titanium tetra-s-butoxide.

7. Process according to Claim 5, **characterized in that** the catalyst used is aluminium oxide, titanium dioxide, titanium tetra-n-propoxide or titanium tetra-i-propoxide.

## Revendications

1. Procédé de préparation d'oxadiazoles substituées de formule générale (I) : 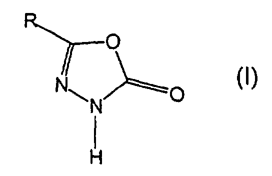 dans laquelle :
R représente un radical alcoyle ayant 1 à 6 atomes de carbone, linéaire ou ramifié, facultativement substitué par un atome d'halogène ou un radical alcoxy en C₁-C₄,
**caractérisé en ce que** l'on fait réagir dans une première étape, un acide carboxylique de formule générale (II) :
R-COOH (II)
dans laquelle:
R a la signification donnée ci-dessus,
avec de l'hydrate d'hydrazine en présence d'un catalyseur et facultativement, en présence d'un agent inerte de dilution et en l'absence d'un alcool, à des températures allant de 0°C à 150°C avec élimination de l'eau, et l'hydrazide d'acide carboxylique formé de formule générale (III) :
R-CO-NH-NH₂ (III)
dans laquelle:
R a la signification donnée ci-dessus,
est mis à réagir dans une deuxième étape, dans le même milieu de réaction, à savoir sans isolement intermédiaire, avec du phosgène (COCl₂) à des températures allant de 20°C à 120°C.

2. Procédé suivant la revendication 1, **caractérisé en ce que** dans les formules (I), (II) et (III), le reste R représente méthyle, éthyle, n- ou i-propyle, n-, i-, s- ou t-butyle, chaque fois facultativement substitué par fluor, chlore, méthoxy ou éthoxy.

3. Procédé suivant la revendication 1, **caractérisé en ce que** l'on travaille dans la première étape du procédé, à des températures allant de 80°C à 130°C.

4. Procédé suivant la revendication 1, **caractérisé en ce que** l'on travaille dans la deuxième étape du procédé, à des températures allant de 40°C à 90°C.

5. Procédé suivant la revendication 1, **caractérisé en ce que** l'on met en oeuvre pour la première étape du procédé, comme catalyseur, l'oxyde d'aluminium, le dioxyde de titane, l'oxyde de zirconium, un alcoolate ou alcoolate d'halogénure d'aluminium, de titane ou de zirconium.

6. Procédé suivant la revendication 5, **caractérisé en ce que** l'on met en oeuvre comme catalyseur, le tri-i-propylate d'aluminium, le tétraméthylate de titane, le tétraéthylate de titane, le tétra-n-propylate de titane, le tétra-i-propylate de titane, le tétra-n-butylate de titane, le tétra-i-butylate de titane ou le tétra-s-butylate de titane.

7. Procédé suivant la revendication 5, **caractérisé en ce que** l'on met en oeuvre comme catalyseur, l'oxyde d'aluminium, le dioxyde de titane, le tétra-n-propylate de titane ou le tétra-i-propylate de titane.
